# FASCICULE DE BREVET EUROPEEN

(11) **EP 3 752 029 B1**
(45) Date de publication et mention de la délivrance du brevet: **12.01.2022**
(21) Numéro de dépôt: 19702936.6
(22) Date de dépôt: 11.02.2019
(51) Int. Cl.: A47F 1/06, A45D 40/04, B65D 83/00, A45D 40/20, A45D 40/00, B65D 83/02, B65D 83/04, A45D 29/00, A45D 34/00, A61F 15/00, A61F 13/15

(54) **DISTRIBUTEUR D'APPLICATEUR CRANTE**
SPENDER MIT GEKERBTEM APPLIKATOR
NOTCHED APPLICATOR DISPENSER

(30) Priorité: 12.02.2018 FR 1800128; 10.07.2018 FR 1800728
(43) Date de publication de la demande: 23.12.2020
(73) Titulaire: Joulia, Pierre, 74290 Talloires-Montmin (FR)
(72) Inventeur: Joulia, Pierre, 74290 Talloires-Montmin (FR)
(74) Mandataire: Cabinet Laurent & Charras
(86) Numéro de dépôt international: PCT/EP2019/053320
(87) Numéro de publication internationale: WO 2019/155063

(56) Documents cités:
- WO-A2-2008/066377
- US-A- 1 973 903
- US-A1- 2017 225 202

## Description

La présente invention a pour objet un distributeur d'applicateurs unidose de retouche, correction et application pour tout domaine et avantageusement destiné à la pharmacie, parapharmacie et cosmétique. Chaque applicateur est destiné à être utilisé une seule fois dans le but d'une parfaite hygiène.

Le but de la nouveauté concerne un dispositif qui garantit une parfaite protection et distribution unitaire d'applicateur et qui pour des raisons notamment d'écologie et de prix de revient contient, tel un réservoir, un nombre maximum d'applicateurs pour un encombrement minimum tout en respectant une ergonomie sécurisante semblable à l'utilisation d'un crayon de maquillage ou d'un tube de rouge à lèvres par exemple.

Dans l'état de la technique actuelle ce sont des produits standards tels que par exemple des tubes de rouge à lèvres, mascara et autres qui sont utilisés comme testeurs dans les magasins ou encore dans les écoles de maquillage et ce type de produit n'étant pas unidose ne présente aucune garantie d'hygiène dans ces cas d'utilisation. De simples spatules ou cotontiges sont parfois utilisés en magasin par frottement sur des produits cosmétiques standards en termes de testeurs mais ce genre d'opération ne permet pas des retouches, corrections ou encore une application précise du produit cosmétique et ne représente donc pas une démonstration ou utilisation du produit en véritable situation. Un distributeur d' applicateurs unidose de retouche correction et application est par exemple connu par le document US2017225202.

La présente invention, qui est définie par les caractéristiques de la revendication 1, offre un service particulièrement adapté en utilisation de testeurs en magasins, dans les écoles d'esthéticiennes ou encore pour une utilisation familiale par exemple.

L'applicateur présente sur sa partie supérieure une face extérieure de retouche, correction et application qui est de forme adaptée suivant les services demandés. Cette première version présente des applicateurs destinés à être frottés ou enduits par l'utilisateur de produit à appliquer sur la peau par exemple pour le médical ou la cosmétique ou sur tout autre support selon les domaines d'utilisation.

Pour une bonne précision d'utilisation, notamment de retouche et correction l'applicateur est conique sur sa partie supérieure.

Le distributeur est particulièrement adapté à une réalisation en mono matière tel que par exemple en polyéthylène ou en polypropylène pour bénéficier très avantageusement d'un recyclage sans démontage.

Dans une version complémentaire l'applicateur est recouvert sur sa face extérieure supérieure d'un flocage pour les retouches, correction et applications.

Dans une version de soins destinés au milieu médical, pharmacie et parapharmacie, l'applicateur est pré-imprégné de produits traitants à utiliser par application tels que par exemple les sérums, les crèmes et pommades traitantes et les pommades labiales.

Dans cette même version le distributeur d'applicateurs est également particulièrement adapté au domaine du maquillage avec des produits cosmétiques tels que de la pâte de rouge à lèvres, du mascara ou eye-liner pour les cils, poudre pour fard et ombres à paupières, parfum solide et tout autre produit de consistance similaire.

Dans une version plus sophistiquée pour résoudre des problèmes d'adhérence du produit cosmétique ou autre à enduire sur l'applicateur, ce dernier est recouvert sur sa face extérieure supérieure d'un flocage, le flocage est pré-imprégné de produits à utiliser par application.

Ainsi, le distributeur d'applicateurs unidose de retouche, de correction et d'application selon l'invention, comprend un poussoir destiné à supporter un lot d'applicateurs unidose empilables, le poussoir étant enchâssé dans un corps supérieur creux qui est enchâssé dans un corps périphérique, le corps périphérique possède une liberté longitudinale par rapport au corps supérieur, le poussoir comprenant sur sa périphérie extérieure au moins une première saillie et au moins une seconde saillie, les saillies sont rendu souples et élastiques par des échancrures latérales, le corps supérieur possède des logements internes latéraux superposés selon un pas régulier, propres à collaborer avec la première saillie, ledit corps supérieur comprend au moins une échancrure longitudinale propres à collaborer avec la seconde saillie, le corps périphérique possède des logements internes latéraux superposés selon un pas identique à celui des logements internes latéraux, propres à collaborer avec la seconde saillie, le corps périphérique comprend un logement interne latéral, le corps supérieur comprend une saillie latérale, le logement interne latéral collabore avec la saillie latérale, le corps périphérique qui est libre longitudinalement en aller-retour par rapport au corps supérieur entraîne le poussoir et les applicateurs dans un déplacement longitudinal vers l'extrémité haute du distributeur.

Selon une caractéristique, la première saillie et la seconde saillie comprennent chacune une face plane inférieure et une face supérieure angulaire.

Selon un mode de réalisation, le corps supérieur est formé sur son extrémité haute d'un jonc intérieur et d'un jonc extérieur, l'au moins une échancrure longitudinale latérale étant située sous le jonc et débouchant sur l'extrémité basse du corps supérieur.

Selon le mode de réalisation précédent, le corps supérieur comprend au moins une échancrure latérale longitudinale débouchant sur son extrémité haute, destinée à donner de la souplesse à la déformation élastique du jonc intérieur.

Selon le mode de réalisation précédent, le corps périphérique est formé à son extrémité supérieure d'au moins un élément ressort qui s'appuie sur la face inférieure horizontale du jonc.

Selon une caractéristique complémentaire, la saillie latérale du corps supérieur comprend une face plane supérieure horizontale et perpendiculaire à l'échancrure longitudinale.

Selon un mode de réalisation, le corps périphérique est fermé à son extrémité inférieure d'une embase qui est formée sur sa périphérie intérieure d'un jonc complémentaire d'une gorge extérieure située à l'extrémité basse du corps périphérique.

Selon le mode de réalisation précédent, le distributeur comprend un corps extérieur dans lequel est enchâssé le corps supérieur, le corps extérieur comprend une gorge intérieure qui collabore en liaison longitudinale avec le jonc du corps supérieur et qui prend appui à son extrémité haute sur la face inférieure horizontale du jonc précité.

Selon le mode de réalisation précédent, l'embase est emmanchée sur sa partie supérieure dans le corps extérieur et collabore de façon étanche avec au moins un jonc intérieur du corps extérieur.

Selon un mode d'exécution, le distributeur comprend une embase comprenant à son extrémité haute, au moins un élément ressort, ladite embase étant emmanchée autour d'au moins l'extrémité inférieure du corps périphérique.

Selon un autre mode d'exécution, l'embase est enchâssé dans un logement du corps extérieur et collabore en butée sur un trottoir supérieur.

D'autres caractéristiques et avantages de l'invention se dégageront de la description qui va suivre en regard des dessins annexés qui ne sont donnés qu'à titre d'exemples non limitatifs.

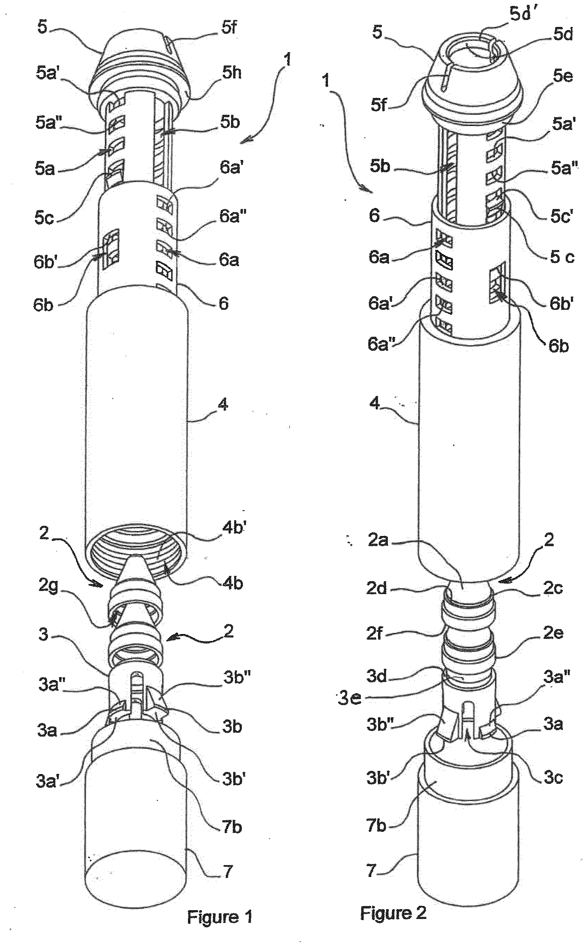

La figure 1 représente une vue éclatée de dessous du distributeur (1).
La figure 2 représente une vue éclatée de dessus du distributeur (1).

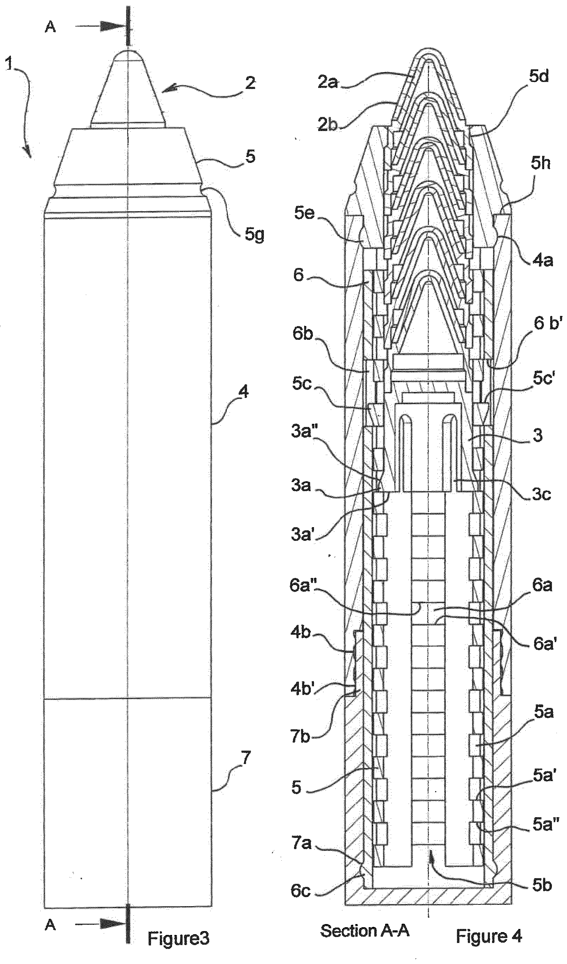

Les figures 3 et 4 représentent le distributeur (1) en vue de face et section AA.

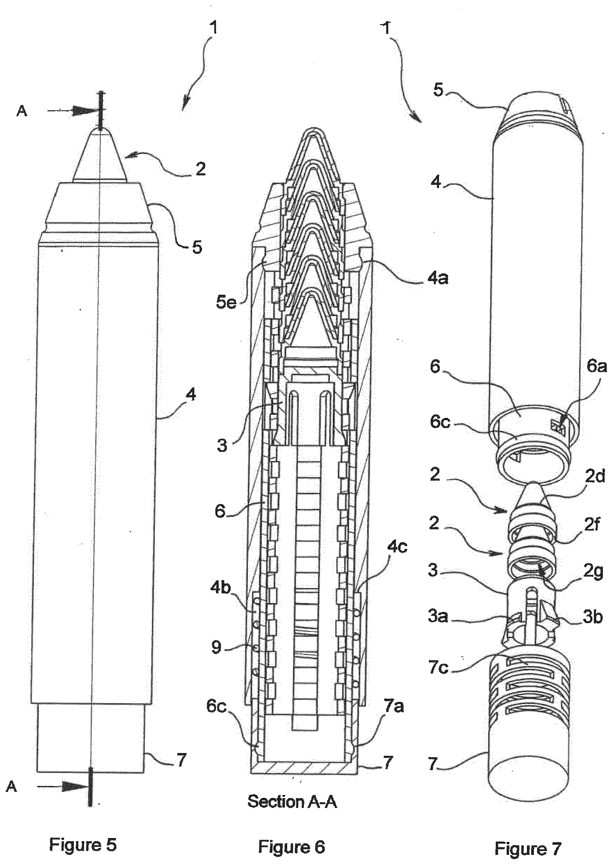

Les figures 5 et 6 représentent une vue de face et une section AA du distributeur (1) selon une version dont l'embase (7) est surmontée d'un élément ressort (9) rapporté.
La figure 7 représente une vue éclatée en perspective du distributeur (1) dont l'embase (7) est prolongée d'un élément ressort (7c).

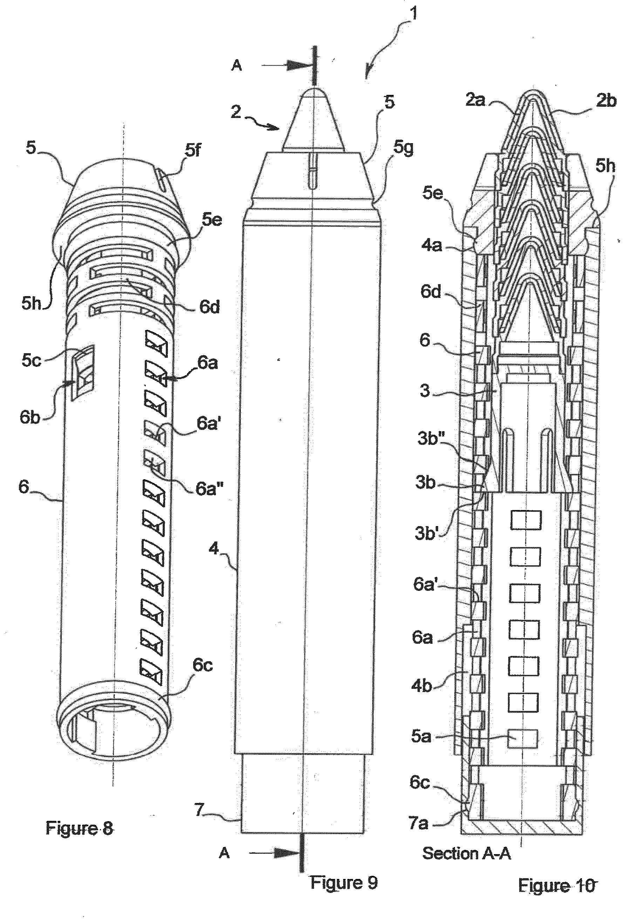

La figure 8 représente en perspective le corps supérieur (5) assemblé avec le corps périphérique (6) prolongée d'un élément ressort (6d).
Les figures 9 et 10 représentent en complément de la figure (8) une vue de face ainsi qu'une section AA du distributeur (1).

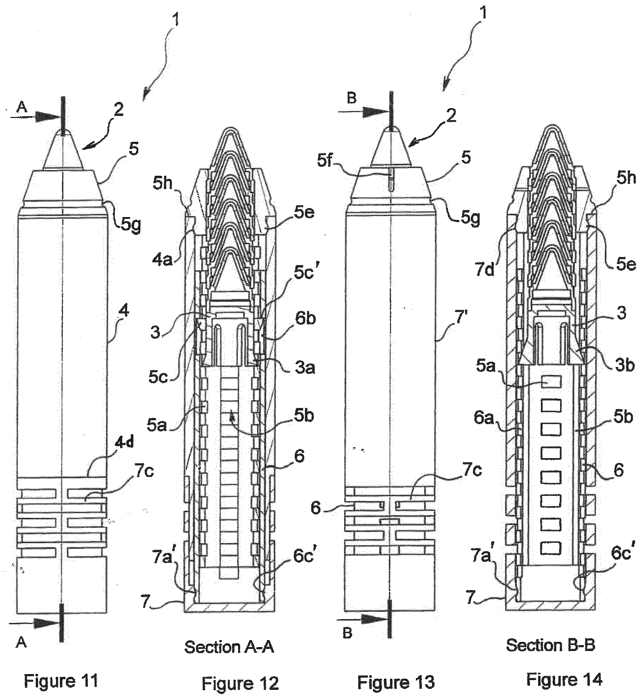

Les figures 11 et 12 représentent une vue de face ainsi qu'une section AA du distributeur (1) dont l'embase (7) est prolongée d'un élément ressort (7c) extérieur.
Les figures 13 et 14 représentent une vue de face ainsi qu'une section BB du distributeur (1) dont l'élément ressort (7c) extérieur est prolongée d'une zone supérieure (7').

La présente invention a pour objet un distributeur (1) d'applicateurs (2) unidose de retouche, correction et application pour tout domaine et avantageusement destiné à la pharmacie, parapharmacie et cosmétique. Le distributeur (1) qui présente une ergonomie semblable à un crayon de maquillage ou d'un tube de rouge à lèvres par exemple est constitué principalement d'un corps supérieur (5) creux, d'un corps périphérique (6), d'un poussoir (3) et d'un lot d'applicateurs (2) unidoses empilables.

Chaque applicateur (2) est utilisé une seule fois dans le but d'une parfaite hygiène.

Le corps supérieur (5) creux, le corps périphérique (6) et le poussoir (3) forment des moyens propres à l'utilisation et à l'éjection des applicateurs (2) positionnés par empilage dans le corps supérieur (5).

L'applicateur (2) formé d'un corps creux comporte sur son extérieur une partie supérieure avec une face (2a) de retouche, correction et application qui est de forme adaptée suivant les services demandés, une partie centrale avec un trottoir (2c) suivi d'un second trottoir (2d) et d'une saillie inférieure latérale (2e) qui est formée d'une face inférieure horizontale (2f) et d'un logement central (2g) débouchant sur l'extrémité basse, complémentaire au trottoir (2c).

Les applicateurs (2) sont assemblés entre eux par empilage, pour cela le trottoir (2c) est emmanché dans le logement (2g) avec la face inférieure horizontale (2f) qui collabore en butée avec le trottoir (2d).

Le poussoir (3) est formé d'une face horizontale supérieure (3d) surmontée d'un bossage (3e) et sur sa périphérie extérieure d'au moins une première saillie (3a) offrant une face plane inférieure (3a') et une face supérieure angulaire (3a") ainsi que d'au moins une seconde saillie (3b) offrant une face plane inférieure (3b') et une face supérieure angulaire (3b"), les saillies (3a) et (3b) sont rendues souples et élastiques par des échancrures latérales (3c).

Le corps supérieur (5) creux, débouchant à ses deux extrémités est formé sur son extrémité haute d'un jonc intérieur (5d) déformable de façon élastique de par les caractéristiques du matériau, d'un jonc (5e) extérieur et d'au moins une échancrure longitudinale (5b) latérale située sous le jonc (5e) et débouchant sur l'extrémité basse du corps supérieur (5).

Le corps supérieur (5) est également formé sur sa périphérie extérieure d'au moins une saillie latérale (5c) située au-dessous du jonc (5e) avec une face plane supérieure (5c') horizontale et perpendiculaire à l'échancrure longitudinale (5b).

Dans une variante, le corps supérieur (5) adaptée aux matériaux plus rigides possède au moins une échancrure longitudinale (5f) débouchant sur son extrémité haute destinée à donner de la souplesse à la déformation élastique du jonc (5d).

Le corps supérieur (5) possède des logements internes latéraux (5a) superposés selon un pas régulier, propres à collaborer avec la première saillie (3a) alors que le corps périphérique (6) possède des logements internes latéraux (6a) superposés selon un pas identique à celui des logements internes latéraux (5a), propres à collaborer avec la seconde saillie (3b).

Le logement interne latéral (6a) possède une face supérieure (6a") et une face inférieure (6a') alors que le logement interne latéral (5a) possède une face supérieure (5a") et une face inférieure (5a').

Les faces (6a"), (6a'), (5a") et (5a') sont sensiblement horizontales et perpendiculaires à l'échancrure longitudinale (5b).

Le corps supérieur (5) est emmanché dans le corps périphérique (6) qui possède une liberté longitudinale par rapport au corps supérieur (5) et les applicateurs (2) sont emmanchés par empilage dans le corps supérieur (5) ainsi que le poussoir (3) par l'orifice de son extrémité inférieure.

Le poussoir (3) qui est réalisé avec un matériau aux caractéristiques élastiques est emmanché dans le corps supérieur (5) avec la seconde saillie (3b) circulant dans l'échancrure longitudinale (5b) et à cet effet le poussoir (3) est emmanché dans l'applicateur (2) avec le bossage supérieur (3e) qui est complémentaire du logement (2g) et dont la face horizontale supérieure (3d) collabore en buté avec la face inférieure horizontale (2f).

Le corps périphérique (6) possède une liberté longitudinale par rapport au corps supérieur (5) égale au pas des logements internes latéraux (6a) ou encore (5a), nécessaire à l'éjection de l'applicateur (2) usagé et pour cela le corps périphérique (6) est formé d'un logement interne latéral (6b) dont la face supérieure (6b') horizontale collabore en butée avec la face plane supérieure (5c') de la saillie latérale (5c).

Le corps périphérique (6) est indexé angulairement par rapport au corps supérieur (5) de par la saillie latérale (5c) qui collabore avec le logement interne latéral (6b).

Le corps périphérique (6) qui est libre longitudinalement en aller-retour par rapport au corps supérieur (5) entraîne le poussoir (3) et les capsules (2) dans un déplacement longitudinal vers l'extrémité haute du distributeur (1) selon la course nécessaire à l'éjection de l'applicateur (2) usagé dont la saillie (2e) force le jonc intérieur (5d) par déformation élastique. Le jonc intérieur (5d) reprend sa forme et dimension initiale et collabore tel un ressort par sa face (5d') chanfreinée avec la face inférieure (2f) de façon à éjecter l'applicateur (2) usagé.

Le trottoir (2d) de l'applicateur (2) suivant, mis en place pour une nouvelle utilisation collabore sur sa périphérie avec le jonc intérieur (5d) par serrage élastique tel un arrêt longitudinal de l'applicateur (2) par rapport au corps supérieur (5) en position d'utilisation.

Pour une immobilisation sécurisée de l'applicateur (2) dans sa position d'utilisation, la saillie (2e) collabore tel un arrêt longitudinal avec le jonc intérieur (5d).

Le corps supérieur (5) possède au moins une échancrure latérale longitudinale (5f) débouchant sur son extrémité haute, destinée à donner de la souplesse à la déformation élastique du jonc intérieur (5d).

Pour cela, lors du déplacement du corps périphérique (6) vers le bas du distributeur (1) la face supérieure (6a") exerce par frottement une pression latérale sur la face supérieure angulaire (3b") de la seconde saillie (3b) et contraint la seconde saillie (3b) qui s'escamote dans le centre du poussoir (3) alors que la face plane inférieure (3a') de la première saille (3a) collabore en butée avec la face (5a') de façon à immobiliser longitudinalement le poussoir (3) par rapport au corps supérieur (5).

Lorsque le corps périphérique (6) est en fin de course dans sa position base, la seconde saillie (3b), qui se trouve positionnée en face du logement interne latéral (6a) supérieur, reprend sa forme initiale de par les caractéristiques élastiques du matériau et coopère avec le dit logement (6a) de par la face plane inférieure (3b') qui collabore en butée avec la face (6a').

Lors du retour du corps périphérique (6) vers le haut du distributeur (1), la face (6a') qui collabore en butée avec la face plane inférieure (3b') entraîne le poussoir (3) vers le haut du distributeur (1) alors que la face (5a") du corps supérieur (5) collabore par frottement avec la face supérieure angulaire (3a") et exerce une pression latérale sur la première saillie (3a) qui s'escamote dans le centre du poussoir (3).

Lorsque le corps périphérique (6) est en fin de course dans sa position haute, la première saillie (3a), qui se trouve positionnée en face du logement interne latéral (5a) supérieur, reprend sa forme initiale de par les caractéristiques élastiques du matériau et coopère avec le dit logement (5a) de par la face plane inférieure (3a') qui collabore en butée avec la face (5a').

Dans une variante de conception le corps périphérique (6) est formé à son extrémité supérieure d'au moins un élément ressort (6d) souple et élastique de par les caractéristiques mécaniques du matériau, qui s'appuie sur la face inférieure horizontale du jonc (5e).

Il suffit alors de pousser le corps périphérique (6) vers le haut du distributeur (1) et de laisser revenir le dit corps périphérique (6) pour que le poussoir (3) se déplace longitudinalement dans le corps supérieur (5) en direction de l'extrémité haute du distributeur (1) selon la course nécessaire à l'éjection de l'applicateur (2) usagé.

Lors du déplacement du corps périphérique (6) vers le haut du distributeur (1) la face (6a') collabore en butée avec la face plane inférieure (3b') et le poussoir (3) est entraîné vers le haut du distributeur (1) alors que la face (5a") du corps supérieur (5) collabore avec la face supérieure angulaire (3a") par frottement et exerce une pression latérale sur la première saillie (3a) qui sous la contrainte s'escamote dans le centre du poussoir (3).

Lorsque le corps périphérique (6) est en fin de course dans sa position haute avec l'élément ressort (6d) compressé, la première saillie (3a) reprend sa forme initiale et coopère avec le logement interne latéral (5a) supérieur avec sa face plane inférieure (3a') qui collabore en butée avec la face (5a').

Au retour du corps périphérique (6) vers le bas du dispositif (1) de par l'action de l'élément ressort (6d), la face supérieure (6a") collabore avec la face supérieure angulaire (3a") par frottement et exerce une pression latérale sur la seconde saillie (3b) qui sous la contrainte s'escamote dans le centre du poussoir (3), alors que la face plane inférieure (3a') de la première saillie (3a) collabore en butée avec la face (5a') de façon à immobiliser longitudinalement le poussoir (3) par rapport au corps supérieur (5). Lorsque le corps périphérique (6) est en fin de course dans sa position basse, la seconde saillie (3b) qui se trouve positionnée en face du logement interne latéral (6a) supérieur reprend sa forme initiale et coopère avec le dit logement (6a) en immobilisation longitudinale de par sa face plane inférieure (3b') qui collabore en butée avec la face (6a').

Afin de proposer une bonne finition extérieure de distributeur (1) le corps périphérique (6) est fermé à son extrémité inférieure d'une embase (7) qui est formée sur sa périphérie intérieure d'un jonc (7a) complémentaire d'une gorge (6c) extérieure située à l'extrémité basse du corps périphérique (6).

Pour une protection extérieure optimisée du distributeur (1) le corps supérieur (5) est emmanché dans un corps extérieur (4) de protection, formé d'une gorge intérieure supérieure (4a) qui collabore en liaison longitudinale avec le jonc (5e) et qui prend appui à son extrémité haute sur la face inférieure horizontale (5h) située au-dessus du jonc (5e).

Dans une version étanche destinée aux applicateurs (2) pré-imprégnés de produits cosmétiques, pharmaceutique ou autres sensible à l'évaporation, l'embase (7) est emmanchée sur sa partie supérieure (7b) dans le corps extérieur (4) et collabore de façon étanche avec au moins un jonc (4b') intérieur.

Dans cette même configuration ergonomique adaptée à une utilisation avec une seule main mais d'une construction différente, l'embase (7) est surmontée sur son extrémité haute d'au moins un élément ressort (9) souple et élastique de par les caractéristiques du matériau, en métal par exemple pour un ressort à spirales ou autres matériaux tels que les matières plastiques adaptées comme par exemple en mousse polyoléfine PE ou PP, les mousses polyuréthanes, les PBT ou encore en résine acétal tel que le polyoxyméthylène ou POM. L'élément ressort (9) est emmanché dans un logement (4b) du corps extérieur (4) et collabore en butée sur un trottoir supérieur (4c).

Dans une variante de conception favorable au recyclage du dispositif (1) de par l'absence de composants métalliques ou encore de divers matériaux non compatibles au recyclage, l'embase (7) est formée sur son extrémité haute d'au moins un élément ressort (7c) souple et élastique de par les caractéristiques du matériau qui est emmanché dans un logement (4b) du corps extérieur (4) et qui collabore en butée sur un trottoir supérieur (4c).

Cette version permet également de tenir le distributeur (1) d'une seule main et d'exercer une pression avec le pouce par exemple sur l'embase (7) pour éjecter l'applicateur (2) usagé, l'embase (7) revient ensuite dans sa position initiale de par la contrainte effectuée par l'élément ressort (9) ou encore (7c) sur le trottoir supérieur (4c).

Dans une variante de réalisation l'élément ressort (7c) est extérieur et prend appui sur un trottoir extérieur (4d).

Dans une version simplifiée faisant l'économie du corps extérieur (4), l'embase (7) est rallongée et l'élément ressort (7c) qui est extérieur prend appui sur le trottoir extérieur (5h) du corps supérieur (5), ou encore pour une manipulation plus aisée bénéficiant de plus d'espace sur le haut de l'élément ressort (7c), ce dernier est surmonté d'une zone supérieure (7') formée d'une gorge intérieure (7d) qui collabore avec le jonc (5e) en arrêt longitudinal. Ainsi l'embase (7) rallongée remplace le corps extérieur (4) et une économie conséquente d'investissement sur le moule et le moulage est réalisée de par la suppression du corps extérieur (4).

Les logements internes latéraux (5a), (6a) et (6b) situés en contre dépouilles internes sont réalisables par des technologies telles que les trois dimensions appelée plus couramment 3d ou stéréo lithographie. Adaptés aux prototypes et petites séries ces technologies ne conviennent pas aux grandes séries et pour cela les logements internes latéraux (5a), (6a) et (6b) sont traversants, réalisés en moulage par injection des matériaux plastiques avec un moule d'injection à chariots extérieur ou encore à tiroirs, le démoulage des logements (5a), (6a) et (6b) se faisant ainsi par l'extérieur du corps supérieur (5) et du corps inférieur (6).

Le conditionnement des applicateurs (2) et du poussoir (3) s'effectue par l'orifice inférieur du corps supérieur (5) qui est préalablement assemblé avec le corps périphérique (6). Le corps extérieur (4) est assemblé sur le corps supérieur (5) et l'embase (7) assemblée sur le corps périphérique (6).

Le corps supérieur (5) est formé sur sa partie supérieure extérieure d'une gorge (5g) destinée à collaborer en immobilisation longitudinale et de façon étanche avec un jonc complémentaire appartenant au capot de protection. Pour une première utilisation, le distributeur (1) libéré du capot de protection est directement utilisable avec un applicateur (2) positionné en mode d'utilisation. Après l'utilisation il est nécessaire d'effectuer un déplacement longitudinal en aller-retour du corps périphérique (6) solidaire de l'embase (7) suivant les versions, pour que l'applicateur (2) usagé soit éjecté du distributeur (1) et remplacé par l'applicateur (2) suivant, pour une future utilisation.

Dans un complément de gamme et pour des produits adaptés à des utilisations spécifiques l'applicateur (2) est recouvert sur sa face extérieure (2a) d'un flocage (2b) adapté à des opérations de retouche, correction et application sur du maquillage par exemple.

En version unidose l'applicateur (2) est pré-imprégné ou recouvert sur sa face extérieure (2a) de produits à utiliser par application, notamment pour les domaines de la pharmacie et parapharmacie avec les pommades labiales, les crèmes et pommades traitantes ou encore pour le maquillage avec des produits cosmétiques tels de la pâte de rouge à lèvres, mascara ou eye-liner pour les cils, poudre pour fard et ombres à paupières, parfum solide et tout autre produit de consistance similaire.

Dans une version plus sophistiquée le flocage (2b) est pré-imprégné de produits à utiliser par application.

Dans une solution particulièrement adaptée à une variante de distributeur (1) rechargeable en applicateurs (2), le poussoir (3) est supprimé au bénéfice de l'applicateur (2) qui intègre les fonctions du dit poussoir et pour cela l'applicateur (2) est formé dans la partie basse de sa périphérie extérieure d'au moins une saillie complémentaire des logements internes latéraux (5a), offrant une face plane inférieure qui collabore en butée avec la face plane inférieure (5a') et une face supérieure angulaire qui collabore par frottement avec la face supérieure (5a") ainsi que d'au moins une saillie complémentaire des logements internes latéraux (6a), offrant une face plane inférieure qui collabore en butée avec la face plane inférieure (6a') et une face supérieure angulaire qui collabore par frottement avec la face supérieure (6a"), les dites saillies complémentaire des logements internes latéraux (5a) et (6a) sont rendu souples et élastiques par des échancrures latérales (2j) débouchant à l'extrémité inférieure de l'applicateur (2) et s'escamotent vers le centre de l'applicateur (2) lors de l'éjection de ce dernier du distributeur (1).

## Revendications

1. Distributeur (1) d'applicateurs (2) unidose de retouche, de correction et d'application comprenant un poussoir (3) destiné à supporter un lot d'applicateurs (2) unidose empilables, le poussoir (3) étant enchâssé dans un corps supérieur (5) creux qui est enchâssé dans un corps périphérique (6), le corps périphérique (6) possède une liberté longitudinale par rapport au corps supérieur (5), le poussoir (3) comprenant sur sa périphérie extérieure au moins une première saillie (3a) et au moins une seconde saillie (3b), les saillies (3a, 3b) sont rendu souples et élastiques par des échancrures latérales (3c), le corps supérieur (5) possède des logements internes latéraux (5a) superposés selon un pas régulier, propres à collaborer avec la première saillie (3a), ledit corps supérieur (5) comprend au moins une échancrure longitudinale (5b) propres à collaborer avec la seconde saillie (3b), le corps périphérique (6) possède des logements internes latéraux (6a) superposés selon un pas identique à celui des logements internes latéraux (5a), propres à collaborer avec la seconde saillie (3b), le corps périphérique (6) comprend un logement interne latéral (6b), le corps supérieur (5) comprend une saillie latérale (5c), le logement interne latéral (6b) collabore avec la saillie latérale (5c), le corps périphérique (6) qui est libre longitudinalement en aller-retour par rapport au corps supérieur (5) entraîne le poussoir (3) et les applicateurs (2) dans un déplacement longitudinal vers l'extrémité haute du distributeur (1).

2. Distributeur (1) selon la revendication 1, **caractérisé en ce que** la première saillie (3a) et la seconde saillie (3b) comprennent chacune une face plane inférieure (3a', 3b') et une face supérieure angulaire (3a", 3b").

3. Distributeur (1) selon la revendication 1 ou 2, **caractérisé en ce que** le corps supérieur (5) est formé sur son extrémité haute d'un jonc intérieur (5d) et d'un jonc (5e) extérieur, l'au moins une échancrure longitudinale (5b) latérale étant située sous le jonc (5e) et débouchant sur l'extrémité basse du corps supérieur (5).

4. Distributeur (1) selon la revendication précédente, **caractérisé en ce que** le corps supérieur (5) comprend au moins une échancrure latérale longitudinale (5f) débouchant sur son extrémité haute, destinée à donner de la souplesse à la déformation élastique du jonc intérieur (5d).

5. Distributeur (1) selon la revendication 3 ou 4, **caractérisé en ce que** le corps périphérique (6) est formé à son extrémité supérieure d'au moins un élément ressort (6d) qui s'appuie sur la face inférieure horizontale du jonc (5e).

6. Distributeur (1) selon l'une quelconque des revendications précédentes, **caractérisé en ce que** la saillie latérale (5c) comprend une face plane supérieure (5c') horizontale et perpendiculaire à l'échancrure longitudinale (5b).

7. Distributeur (1) selon l'une quelconque des revendications précédentes, **caractérisé en ce que** le corps périphérique (6) est fermé à son extrémité inférieure d'une embase (7) qui est formée sur sa périphérie intérieure d'un jonc (7a) complémentaire d'une gorge (6c) extérieure située à l'extrémité basse du corps périphérique (6).

8. Distributeur (1) selon la revendication précédente, **caractérisé en ce qu'**il comprend un corps extérieur (4) dans lequel est enchâssé le corps supérieur (5), le corps extérieur (4) comprend une gorge intérieure (4a) qui collabore en liaison longitudinale avec le jonc (5e) et qui prend appui à son extrémité haute sur la face inférieure horizontale (5h) du jonc (5e).

9. Distributeur (1) selon la revendication 7 et 8, **caractérisé en ce que** l'embase (7) est emmanchée sur sa partie supérieure (7b) dans le corps extérieur (4) et collabore de façon étanche avec au moins un jonc (4b') intérieur du corps extérieur (4).

10. Distributeur (1) selon l'une quelconque des revendications 1 à 6, **caractérisé en ce qu'**il comprend une embase (7) comprenant à son extrémité haute, au moins un élément ressort (7c), ladite embase (7) étant emmanchée autour d'au moins l'extrémité inférieure du corps périphérique (6).

11. Distributeur (1) selon l'une quelconque des revendications 7 à 10, **caractérisé en ce que** l'embase (7) est enchâssé dans un logement (4b) du corps extérieur (4) et collabore en butée sur un trottoir supérieur (4c).

## Patentansprüche

1. Spender (1) von Einzeldosisapplikatoren (2) zur Retusche, Korrektur und Applikation, umfassend einen Stößel (3), der dazu bestimmt ist, einen Satz von stapelbaren Einzeldosisapplikatoren (2) zu tragen, wobei der Stößel (3) in einem hohlen, oberen Körper (5) eingefügt ist, der in einem Umfangskörper (6) eingefügt ist, der Umfangskörper (6) besitzt eine Längsfreiheit in Bezug auf den oberen Körper (5), wobei der Stößel (3) auf seinem Außenumfang wenigstens einen ersten Vorsprung (3a) und wenigstens einen zweiten Vorsprung (3b) umfasst, die Vorsprünge (3a, 3b) werden durch seitliche Aussparungen (3c) biegsam und elastisch gemacht, der obere Körper (5) besitzt seitliche Innenaufnahmen (5a), die gemäß einer regelmäßigen Teilung übereinanderliegend sind, geeignet um mit dem ersten Vorsprung (3a) zusammenzuwirken, der obere Körper (5) umfasst wenigstens eine Längsaussparung (5b), geeignet um mit dem zweiten Vorsprung (3b) zusammenzuwirken, der Umfangskörper (6) besitzt seitliche Innenaufnahmen (6a), die gemäß einer Teilung, die mit jener der seitlichen Innenaufnahmen (5a) identisch ist die übereinanderliegend sind, geeignet um mit dem zweiten Vorsprung (3b) zusammenzuwirken, der Umfangskörper (6) umfasst eine seitliche Innenaufnahme (6b), der obere Körper (5) umfasst einen seitlichen Vorsprung (5c), die seitliche Innenaufnahme (6b) wirkt mit dem seitlichen Vorsprung (5c) zusammen, der Umfangskörper (6), der in Bezug auf den oberen Körper (5) hin und zurück der Länge nach frei ist, nimmt den Stößel (3) und die Applikatoren (2) in einer Längsverschiebung zum oberen Ende des Spenders (1) mit.

2. Spender (1) nach Anspruch 1, **dadurch gekennzeichnet, dass** der erste Vorsprung (3a) und der zweite Vorsprung (3b) jeweils eine untere ebene Fläche (3a`, 3b`) und eine obere gewinkelte Fläche (3a", 3b") umfassen.

3. Spender (1) nach Anspruch 1 oder 2, **dadurch gekennzeichnet, dass** der obere Körper (5) auf seinem oberen Ende von einem inneren Reif (5d) und einem äußeren Reif (5e) gebildet wird, wobei sich die wenigstens eine seitliche Längsaussparung (5b) unter dem Reif (5e) befindet und auf das untere Ende des oberen Körpers (5) mündet.

4. Spender (1) nach dem vorstehenden Anspruch, **dadurch gekennzeichnet, dass** der obere Körper (5) wenigstens eine seitliche Längsaussparung (5f) umfasst, die auf sein oberes Ende mündet, dazu bestimmt, dem inneren Reif (5d) Biegsamkeit bei der elastischen Verformung zu verleihen.

5. Spender (1) nach Anspruch 3 oder 4, **dadurch gekennzeichnet, dass** der Umfangskörper (6) an seinem oberen Ende von wenigstens einem Federelement (6d) gebildet wird, das sich auf die horizontale untere Fläche des Reifs (5e) stützt.

6. Spender (1) nach einem der vorstehenden Ansprüche, **dadurch gekennzeichnet, dass** der seitliche Vorsprung (5c) eine obere ebene Fläche (5c`) umfasst, die horizontal und senkrecht zu der Längsaussparung (5b) verläuft.

7. Spender (1) nach einem der vorstehenden Ansprüche, **dadurch gekennzeichnet, dass** der Umfangskörper (6) an seinem unteren Ende von einem Sockel (7) verschlossen wird, der auf seinem Innenumfang von einem Reif (7a) gebildet wird, und komplementär ist zu einer äußeren Nut (6c), die sich am unteren Ende des Umfangskörpers (6) befindet.

8. Spender (1) nach dem vorstehenden Anspruch, **dadurch gekennzeichnet, dass** er einen äußeren Körper (4) umfasst, in dem der obere Körper (5) eingefügt ist, der äußere Körper (4) eine innere Nut (4a) umfasst, die in Längsverbindung mit dem Reif (5e) zusammenwirkt und die an ihrem oberen Ende auf die horizontale untere Fläche (5h) des Reifs (5e) drückt.

9. Spender (1) nach Anspruch 7 und 8, **dadurch gekennzeichnet, dass** der Sockel (7) auf seinem oberen Teil (7b) in den äußeren Körper (4) eingepasst ist und auf dichte Weise mit wenigstens einem inneren Reif (4b`) des äußeren Körpers (4) zusammenwirkt.

10. Spender (1) nach einem der Ansprüche 1 bis 6, **dadurch gekennzeichnet, dass** er einen Sockel (7) umfasst, der an seinem oberen Ende wenigstens ein Federelement (7c) umfasst, wobei der Sockel (7) um wenigstens das untere Ende des Umfangskörpers (6) eingepasst ist.

11. Spender (1) nach einem der Ansprüche 7 bis 10, **dadurch gekennzeichnet, dass** der Sockel (7) in einer Aufnahme (4b) des äußeren Körpers (4) eingefügt ist und anschlagend auf einen oberen Rand (4c) zusammenwirkt.

## Claims

1. Dispenser (1) of single-dose retouching, correction and application applicators (2) comprising a pusher (3) for supporting a batch of stackable single-dose applicators (2), the pusher (3) being embedded in a hollow upper body (5) which is embedded in a peripheral body (6), the peripheral body (6) having longitudinal freedom in relation to the upper body (5), the pusher (3) comprising on its outer periphery at least one first protrusion (3a) and at least one second protrusion (3b), the protrusions (3a, 3b) being made flexible and elastic by lateral notches (3c), the upper body (5) having internal lateral housings (5a) arranged one above the other with a regular pitch, capable of cooperating with the first protrusion (3a), said upper body (5) comprising at least one longitudinal notch (5b) capable of cooperating with the second protrusion (3b), the peripheral body (6) having internal lateral housings (6a) arranged one above the other with a pitch identical to that of the internal lateral housings (5a), capable of cooperating with the second protrusion (3b), the peripheral body (6) comprising an internal lateral housing (6b), the upper body (5) comprising a lateral protrusion (5c), the internal lateral housing (6b) cooperating with the lateral protrusion (5c), the peripheral body (6) which is longitudinally free to move back and forth with respect to the upper body (5) driving the pusher (3) and the applicators (2) in a longitudinal movement towards the upper end of the dispenser (1).

2. Dispenser (1) according to claim 1, **characterised in that** the first protrusion (3a) and the second protrusion (3b) each comprise a lower flat side (3a', 3b') and an upper angular side (3a*"*, 3b*"*).

3. Dispenser (1) according to claim 1 or 2, **characterised in that** the upper body (5) is formed at its upper end by an inner ring (5d) and an outer ring (5e), the at least one lateral longitudinal notch (5b) being located beneath the ring (5e) and opening onto the lower end of the upper body (5).

4. Dispenser (1) according to the preceding claim, **characterised in that** the upper body (5) comprises at least one lateral longitudinal notch (5f) opening out at its upper end, for giving flexibility to the elastic deformation of the inner ring (5d).

5. Dispenser (1) according to claim 3 or 4, **characterised in that** the peripheral body (6) is formed at its upper end by at least one spring element (6d) which bears on the horizontal underside of the ring (5e).

6. Dispenser (1) according to any one of the preceding claims, **characterised in that** the lateral protrusion (5c) comprises an upper flat side (5c') horizontal and perpendicular to the longitudinal notch (5b).

7. Dispenser (1) according to any one of the preceding claims, **characterised in that** the peripheral body (6) is closed at its lower end by a base (7) which is formed on its inner periphery by a ring (7a) in addition to an outer groove (6c) located at the lower end of the peripheral body (6).

8. Dispenser (1) according to the preceding claim, **characterised in that** it comprises an outer body (4) in which the upper body (5) is embedded, the outer body (4) comprising an inner groove (4a) which cooperates longitudinally with the ring (5e) and which bears at its upper end on the horizontal underside (5h) of the ring (5e) .

9. Dispenser (1) according to claim 7 and 8, **characterised in that** the base (7) is fitted on its upper part (7b) into the outer body (4) and cooperates in a sealed manner with at least one inner ring (4b') of the outer body (4).

10. Dispenser (1) according to any one of claims 1 to 6, **characterised in that** it comprises a base (7) comprising at its upper end at least one spring element (7c), said base (7) being fitted around at least the lower end of the peripheral body (6).

11. Dispenser (1) according to any one of claims 7 to 10, **characterised in that** the base (7) is embedded in a housing (4b) of the outer body (4) and cooperates in abutment with an upper ledge (4c).
